# EUROPEAN PATENT APPLICATION

(11) **EP 1 002 779 A1**
(43) Date of publication of application: **24.05.2000**
(21) Application number: 99121692.0
(22) Date of filing: 02.11.1999
(51) Int. Cl.: C07C 7/163

(54) **Process for desulphurisation of a hydrocarbon stream**

(30) Priority: 18.11.1998 US 108977 P
(71) Applicant: Haldor Topsoe A/S, DK-2800 Lyngby (DK)
(72) Inventor: Hansen, Jens-Henrik Bak, 2800 Lyngby (DK); Olsen, Christian, 2750 Ballerup (DK); Smits, Richard, 2800 Lyngby (DK); Christensen, Thomas Sandahl, 2800 Lyngby (DK)

(57) **Abstract**

Process for the improved desulphurization of a hydrocarbon stream being further steam reformed comprising steps of
(a) splitting a stream from the steam reformed hydrocarbon, thus containing hydrogen;
(b) optionally reducing the amount of water from the hydrogen containing split stream;
(c) reinjecting the hydrogen containing split stream, optionally reduced in water in process step (b), to an ejector driven by the hydrocarbon feed stream as motive stream; and
(d) admitting the hydrogen containing hydrocarbon admixture to the hydrogenation step.

## Description

The present invention is directed to desulphurization of a hydrocarbon stream in presence of hydrogen.

In particular, the invention concerns improvements in the desulphurization step of a hydrocarbon stream in a process where hydrogen is not readily available and where the hydrocarbon stream is further processed in a catalytic steam reforming step.

When hydrogen for desulphurization is readily available, the ordinary process route involves addition of hydrogen from the hydrogen source to the hydrocarbon stream before it is preheated and passed on to the desulphurization step comprising hydrogenation of organic sulphur compounds to H₂S and subsequent absorption of H₂S in a sulphur absorber;
steam is added to the desulphurized hydrocarbon stream, the admixture is heated and the hydrocarbon/steam stream is reformed forming primarily hydrogen, carbon monoxide and carbon dioxide as part of the product, leaving part of the methane and steam unconverted. If the hydrocarbon contains a substantial fraction of higher hydrocarbons it may be advantageous to prereform such a hydrocarbon stream in a so called prereformer at a lower temperature than normally applied in a conventional steam reformer. The desulphurization step prior to the reforming step is mandatory in order not to poison the steam reformer catalyst.

Desulphurization of a hydrocarbon stream can be accomplished without hydrogen, however, hydrogen improves the desulphurization process step as to both conversion and catalyst consumption.

Conventionally, when hydrogen is not readily available, hydrogen is provided from the downstream process and recycled to the desulphurization process step by means of a recycle compressor.

It has now been found that the integration of an ejector into the process steps of desulphurization and steam reforming of a hydrocarbon stream may provide for the hydrogen required in a cheap and robust way. An ejector is well known to be highly reliable and simple in construction with no moving parts, thus fabricated at a low cost.
If a small split stream of the reformed hydrocarbon is recycled and lead to an ejector as the suction stream driven by the hydrocarbon feed stream to the hydrogenation reactor as the motive stream, a sufficient amount of hydrogen can be provided for the desulphurization process with only a minor loss of pressure of the hydrocarbon stream. Water is beneficially reduced from the recycle stream prior to being introduced to the ejector as the introduction of water into the hydrocarbon feed reduces the performance of the down stream sulphur absorber.

In accordance with the above finding, this invention provides an improved process for the desulphurization of a hydrocarbon feedstock and subsequent steam reforming of the feedstock, comprising steps of:
splitting a stream from the steam reformed hydrocarbon, thus containing hydrogen;
optionally reducing the amount of water from the hydrogen containing split stream;
re-injecting the hydrogen containing split stream optionally reduced in water to an ejector driven by a stream of hydrocarbon feedstock as motive stream; and
passing the hydrogen containing hydrocarbon admixture to the desulphurization step.

Suitable means of reduction of water is cooling and separation but other processes, which reduce the water content of the split stream may be applied as well.

Even at a high degree of desulphurization of the hydrocarbon feedstock, some sulphur compounds in the hydrocarbon feedstock will reach and come in contact with the steam reforming catalyst, and most likely sulphur compounds will also be introduced with the steam added upstream the steam reforming step. Sulphur compounds lead to catalyst poisoning and loss of activity, which gradually will proceed from the inlet/top towards the exit/bottom of the bed of the steam reforming catalyst, which is typically nickel based. If the sulphur poisoned steam reforming catalyst is subjected to oxidative conditions during operation, the active nickel metal is converted into inactive nickel oxide;
at the same time sulphur is released and is moving further downwards the steam reforming catalyst bed, further poisoning a lower layer of catalyst, in turn resulting in an overall very rapid deterioration of the performance of the steam reforming catalyst.

As a further advantage of the invention the hydrogen will help prevent the oxidation of the sulphur poisoned steam reforming catalyst.

A specific embodiment of the present invention is described in the Example below.

### Example

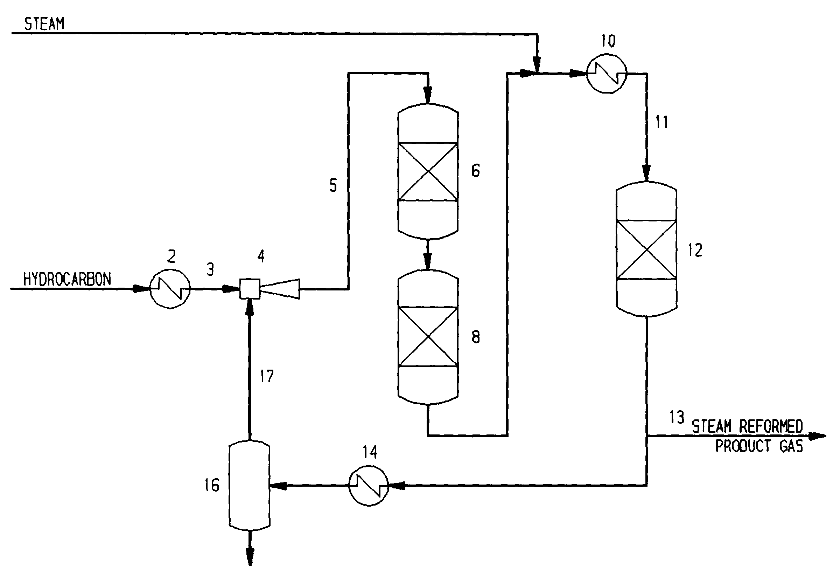

This is a calculation example of the present invention. In the description of the example, reference is made to Fig. 1.

A hydrocarbon feed stream is preheated in one or more heaters **2**, using suitable heat sources; the heated hydrocarbon stream **3** is passed to an ejector **4** as a motive stream, driving a hydrogen containing stream **17**, which is passed to the ejector **4** at a lower pressure.

The effluent **5** from the ejector is passed on to a desulphurization step comprising a hydrogenator **6**, which hydrogenates organic sulphur compounds to H₂S, and thence to a sulphur absorber **8**, which removes H₂S. The desulphurised hydrocarbon stream is mixed with process steam and preheated further in one or more preheaters **10**, the preheated stream **11** being introduced to an adiabatic steam reformer **12**, the so-called prereformer, where it is steam reformed into a synthesis gas stream rich in hydrogen, carbon monoxide, carbon dioxide, methane and steam. The effluent stream from the prereformer is split into the main steam reformed product gas stream **13** and a smaller stream which is cooled in one or more coolers **14** and separated into liquid stream and vapour stream **17** in the separator **16**. The hydrogen containing vapour stream **17** from the separator **16** is, as mentioned above, passed to the ejector **4**.

In Table 1 below is presented the calculation results from the above process scheme.

**Table 1**

| Position | 3 | 5 | 11 | 13 | 17 |
|---|---|---|---|---|---|
| Pressure [kPa] | 2102 | 1916 | 1680 | 1631 | 1563 |
| Temperature [°C] | 397 | 379 | 454 | 401 | 40 |
| Dry Flow rate [kmole/h] | 809.2 | 864.9 | 864.9 | 1000.1 | 55.7 |
| Water flow [kg/h] | 0. | 5. | 34189. | 30756. | 5. |

| Composition [dry mole %] | | | | | |
|---|---|---|---|---|---|
| CH₄ | 81.30 | 80.40 | 80.40 | 67.36 | 67.39 |
| C₂H₆ | 2.85 | 2.67 | 2.67 | | |
| C₃H₈ | 0.37 | 0.35 | 0.35 | | |
| C₄H₁₀ | 0.14 | 0.13 | 0.13 | | |
| C₅H₁₂ | 0.04 | 0.04 | 0.04 | | |
| C₆H₁₄ | 0.05 | 0.05 | 0.05 | | |
| N₂ | 14.36 | 14.18 | 14.18 | 11.62 | 11.63 |
| CO₂ | 0.89 | 1.18 | 1.18 | 5.46 | 5.41 |
| H₂ | | 1.00 | 1.00 | 15.52 | 15.53 |
| CO | | <0.01 | <0.01 | 0.04 | 0.04 |

## Claims

1. Process for the improved desulphurization of a hydrocarbon stream being further steam reformed comprising steps of
(a) splitting a stream from the steam reformed hydrocarbon, thus containing hydrogen;
(b) optionally reducing the amount of water from the hydrogen containing split stream;
(c) reinjecting the hydrogen containing split stream, optionally reduced in water in process step (b), to an ejector driven by the hydrocarbon feed stream as motive stream; and
(d) admitting the hydrogen containing hydrocarbon admixture to the hydrogenation step.

2. The process as recited in claim 1, wherein the optional step (b) of water reduction is performed by cooling and separation.
